(19) Europäisches Patentamt
European Patent Office
Office européen des brevets

(11) Veröffentlichungsnummer: 0 347 684 A1

(12) **EUROPÄISCHE PATENTANMELDUNG**

(21) Anmeldenummer: 89110535.5

(51) Int. Cl.4: C07D 263/46 , A01N 43/76

(22) Anmeldetag: 10.06.89

(30) Priorität: 24.06.88 DE 3821300

(43) Veröffentlichungstag der Anmeldung: 27.12.89 Patentblatt 89/52

(84) Benannte Vertragsstaaten: BE CH DE ES FR GB IT LI NL

(71) Anmelder: BAYER AG
D-5090 Leverkusen 1 Bayerwerk(DE)

(72) Erfinder: Gajowski, Jan, Dr.
Insterburger Strasse 34
D-4019 Monheim(DE)
Erfinder: Naumann, Klaus, Dr.
Richard-Wagner-Strasse 9
D-5090 Leverkusen(DE)
Erfinder: Hartwig, Jürgen, Dr.
Franz-Esser-Strasse 44
D-5090 Leverkusen 3(DE)

(54) **2-(Halogen)alkylthio-1,3-oxazol-Derivate.**

(57) Es werden neue 2-(Halogen)alkylthio-1,3-oxazol-Derivate der Formel

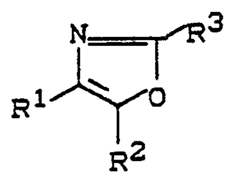

(I)

bereitgestellt,
in welcher
$R^1$ für Wasserstoff, Alkyl oder gegebenenfalls substituiertes Phenyl steht,
$R^2$ für Wasserstoff, Alkyl oder gegebenenfalls substituiertes Phenyl steht und
$R^3$ für gegebenenfalls durch Halogen substituiertes Alkylthio steht,
mit der Maßgabe, daß $R^1$ und $R^2$ voneinander verschieden sind.
Die neuen Verbindungen der Formel (I) besitzen eine stark ausgeprägte Wirksamkeit gegen tierische Schädlinge, insbesondere gegen Insekten.

EP 0 347 684 A1

## 2-(Halogen)alkylthio-1,3-oxazol-Derivate

Die Erfindung betrifft neue 2-(Halogen)alkylthio-1,3-oxazol Derivate, ein Verfahren zu ihrer Herstellung sowie ihre Verwendung in Schädlingsbekämpfungsmitteln, insbesondere als Insektizide.

Es ist bereits bekannt, daß bestimmte N-Alkyl-substituierte Thionophosphorsäureesteramide, wie z.B. O-Ethyl-O-(2-isopropyloxycarbonyl-phenyl)-N-isopropyl-thionophosphorsäureesteramid, zur Bekämpfung von Insekten geeignet sind (vgl. US-PS 2 978 479: DE-OS 16 68 047). Die insektizide Wirkung dieser bekannten Verbindung ist jedoch, insbesondere bei niedrigen Aufwandmengen bzw. Wirkstoffkonzentrationen, nicht immer voll zufriedenstellend.

Es wurden nun die neuen 2-(Halogen)alkylthio-1,3-oxazol-Derivate der Formel

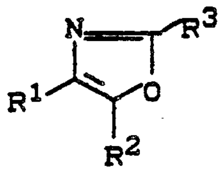

(I)

gefunden,
in welcher
$R^1$ für Wasserstoff, Alkyl oder gegebenenfalls substituiertes Phenyl steht,
$R^2$ für Wasserstoff, Alkyl oder gegebenenfalls substituiertes Phenyl steht und
$R^3$ für gegebenenfalls durch Halogen substituiertes Alkylthio steht,
mit der Maßgabe, daß $R^1$ und $R^2$ voneinander verschieden sind.

Weiterhin wurde gefunden, daß man die neuen 2-(Halogen)alkylthio-1,3-oxazol-Derivate der Formel (I) erhält, wenn man 1,3-Oxazol-Derivate der Formel

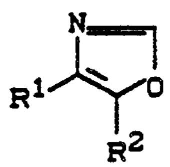

(II)

in welcher
$R^1$ für Wasserstoff, Alkyl oder gegebenenfalls substituiertes Phenyl steht und
$R^2$ für Wasserstoff, Alkyl oder gegebenenfalls substituiertes Phenyl steht,
mit der Maßgabe, daß $R^1$ und $R^2$ voneinander verschieden sind,
in allgemein üblicher Art und Weise mit Lithiumverbindungen der Formel
$R^4$ - Li (III)
in welcher
$R^4$ für Methyl oder n-Butyl, vorzugsweise n-Butyl steht
gegebenenfalls in Gegenwart von Verdünnungsmitteln, gegebenenfalls in Gegenwart eines Inertgases und anschließend mit Sulfenylhalogeniden der Formel
$R^3$ - X (IV)
in welcher
$R^3$ für gegebenenfalls durch Halogen substituiertes Alkylthio steht und
X für Halogen, insbesondere Fluor, Chlor oder Brom, vorzugsweise Chlor steht,
umsetzt.

Schließlich wurde gefunden, daß sich die neuen 2-Halogenalkylthio-1,3-oxazol-Derivate der Formel (I) durch sehr starke insektizide Wirksamkeit auszeichnen.

Die neuen Verbindungen der Formel (I) zeigen insbesondere eine überraschende, hervorragende Wirkung gegen Nematoden, wie z.B. Globodera rostochiensis und Meloidogyne incognita.

Überraschenderweise zeigen die erfindungsgemäßen 2-Halogenalkylthio-1,3-oxazol-Derivate der Formel (I) vor allem eine bessere insektizide Wirkung als die oben erwähnten bekannten N-Alkyl-substituierten Thionophosphorsäureesteramide. Die erfindungsgemäßen Stoffe stellen somit eine wertvolle Bereicherung der Technik dar.

Im folgenden sind in den bevorzugten und besonders bevorzugten Bereichen die bereits in der Hauptdefinition ausgeschlossenen Verbindungen ebenfalls ausgeschlossen.

Die Erfindung betrifft vorzugsweise Verbindungen der Formel (I), in welcher

$R^1$ für Wasserstoff, ($C_1$-$C_4$)-Alkyl oder für gegebenenfalls einfach bis fünffach, gleich oder verschieden durch Fluor, Chlor, Brom, durch ($C_1$-$C_4$)-Alkyl (welches gegebenenfalls durch Fluor und/oder Chlor substituiert ist) oder durch ($C_1$-$C_4$)-Alkoxy substituiertes Phenyl steht,

$R^2$ für Wasserstoff, ($C_1$-$C_4$)-Alkyl oder für gegebenenfalls einfach bis fünffach, gleich oder verschieden durch Fluor, Chlor, Brom, durch ($C_1$-$C_4$)-Alkyl (welches gegebenenfalls durch Fluor und/oder Chlor substituiert ist) oder durch ($C_1$-$C_4$)-Alkoxy substituiertes Phenyl steht und

$R^3$ für gegebenenfalls durch Fluor und/oder Chlor substituiertes ($C_1$-$C_4$)-Alkylthio steht.

Besonders bevorzugt sind diejenigen Verbindungen der Formel (I), in welcher

$R^1$ für Wasserstoff, Methyl, Ethyl oder für gegebenenfalls einfach bis dreifach, gleich oder verschieden durch Fluor und/oder Chlor, durch ($C_1$-$C_2$)-Alkyl (welches gegebenenfalls einfach bis fünffach durch Fluor und/oder Chlor substituiert ist), oder durch Methoxy oder Ethoxy substituiertes Phenyl steht,

$R^2$ für Wasserstoff, Methyl, Ethyl oder für gegebenenfalls einfach bis dreifach, gleich oder verschieden durch Fluor und/oder Chlor, durch ($C_1$-$C_2$)-Alkyl (welches gegebenenfalls einfach bis fünffach durch Fluor und/oder Chlor substituiert ist), oder durch Methoxy oder Ethoxy substituiertes Phenyl steht und

$R^3$ für gegebenenfalls einfach bis fünffach, gleich oder verschieden durch Fluor und/oder Chlor substituiertes ($C_1$-$C_2$)-Alkylthio steht.

Ganz besonders bevorzugt sind diejenigen Verbindungen der Formel (I), in welcher

$R^1$ für Wasserstoff, Methyl oder für gegebenenfalls einfach bis dreifach, gleich oder verschieden durch Fluor und/oder Chlor, Trifluormethyl oder Methoxy substituiertes Phenyl steht,

$R^2$ für Wasserstoff, Methyl oder für gegebenenfalls einfach bis dreifach, gleich oder verschieden durch Fluor und/oder Chlor, Trifluormethyl oder Methoxy substituiertes Phenyl steht und

$R^3$ für Methylthio, Chlordifluormethylthio, Dichlorfluormethylthio oder Trifluormethylthio steht.

Verwendet man beispielsweise für das erfindungsgemäße Verfahren 5-(4′-Fluorphenyl)-1,3-oxazol, n-Butyllithium und Chlordifluormethansulfenylchlorid als Ausgangsstoffe, so kann die entsprechende Reaktion durch das folgende Formelschema wiedergegeben werden:

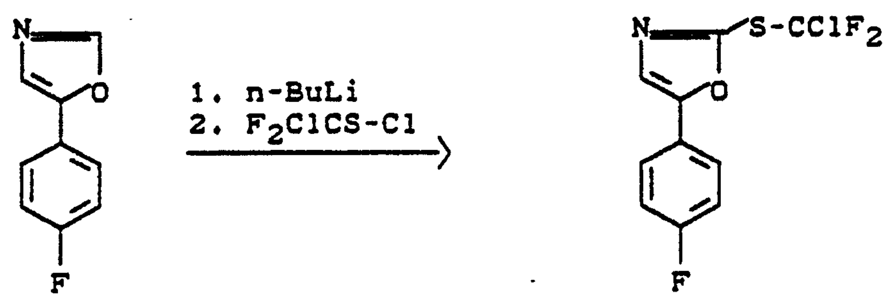


Verwendet man beispielsweise für das erfindungsgemäße Verfahren 4-(2′-Fluorphenyl)-1,3-oxazol, n-Butyllithium und Trifluormethansulfenylchlorid, so kann die entsprechende Reaktion durch das folgende Formelschema wiedergegeben werden:

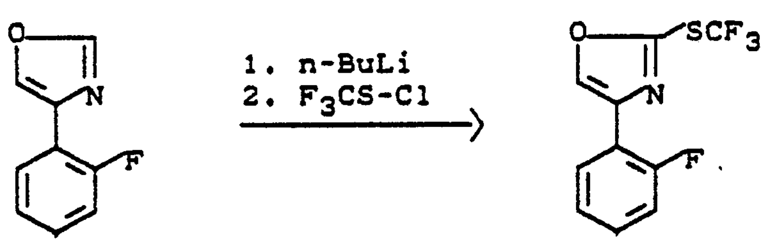


Die beim erfindungsgemäßen Verfahren als Ausgangsstoffe zu verwendenden 1,3-Oxazol-Derivate sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäßen Stoffe der Formel (I) vorzugsweise für diese Substituenten genannt wurden.

Die Verbindungen der Formel (II) sind bekannt und/oder lassen sich nach bekannten Methoden

herstellen [vergl. Tetrahedron Lett., 2367-2372 (1972), Angew. Chem. 83, 358-359 (1971)].

Die bei dem erfindungsgemäßen Verfahren außerdem als Ausgangsstoffe zu verwendenden Lithiumverbindungen der Formel (III) sind allgemein bekannte Verbindungen der organischen Chemie.

Die bei dem erfindungsgemäßen Verfahren außerdem als Ausgangsstoffe zu verwendenden Sulfensäurehalogenide der Formel (IV) sind allgemein bekannte Verbindungen der organischen Chemie. $R^3$ steht in dieser Formel für Halogen, insbesondere Chlor oder Brom, besonders bevorzugt Chlor.

Das erfindungsgemäße Verfahren zur Herstellung der neuen Verbindungen der Formel (I) wird bevorzugt unter Verwendung von Verdünnungsmitteln durchgeführt. Als Verdünnungsmittel kommen praktisch alle organischen inerten Lösungsmittel in Frage.

Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Ether wie Diethyl- und Dibutylether, Glykoldimethylether und Diglycoldimethylether, Tetrahydrofuran und Dioxan und Hexamethylphosphorsäuretriamid.

Das erfindungsgemäße Verfahren wird gegebenenfalls in Gegenwart eines Inertgases durchgeführt. Als Inertgase kommen dabei Stickstoff sowie praktisch alle Edelgase, insbesondere Argon infrage.

Die Reaktionstemperaturen können bei der Durchführung der erfindungsgemäßen Verfahrens in einem größeren Bereich variiert werden. Im allgemeinen arbeitet man bei Temperaturen zwischen -80 °C und +50 °C, vorzugsweise bei Temperaturen zwischen -80 °C und +25 °C. Die Umsetzungen werden im allgemeinen bei Normaldruck durchgeführt.

Zur Durchführung des erfindungsgemäßen Verfahrens setzt man auf 1 Mol 1,3-Oxazol der Formel (II) 1 bis 1,5 Mol, vorzugsweise 1 bis 1,2 Mol Lithiumverbindung der Formel (III) und 1 bis 3, vorzugsweise 1 bis 2 Mol Sulfenylhalogenid der Formel (IV) ein. Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die Wirkstoffe eignen sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes ehrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Die erfindungsgemäßen Wirkstoffe der Formel (I) zeichnen sich durch eine hervorragende insektizide Wirksamkeit aus. So ist insbesondere die hervorragende Wirkung gegen Nematoden, wie z.B. Globodera rostochiensis und Meloidogyne incognita zu nennen.

Einige der erfindungsgemäßen Wirkstoffe zeigen auch blattinsektizide und akarizide Wirkung. Sie können außerdem auch zur Bekämpfung von Hygieneschädlingen eingesetzt werden.

Die Wirkstoffe können in die üblichen Formulierungen übergeführt werden wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aro maten oder chlorierte al phatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können. Sie zeigen insbesondere beim Einsatz als Ektoparasitizide eine ausgezeichnete Wirkung gegen Blowfly-larven wie z. B. Lucilia cuprina.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns.

Die biologische Wirksamkeit der erfindungsgemäßen Verbindungen soll anhand der folgenden Beispiele erläutert werden:

## Herstellungsbeispiele

### Beispiel 1

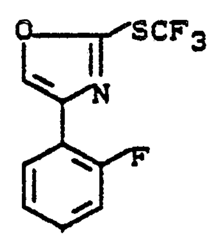

Unter Argon-Atmosphäre läßt man zur Lösung von 5,0 g (31 mMol) 4-(2′-Fluorphenyl)-1,3-oxazol in 100 ml wasserfreiem Ether bei -78 °C unter Rühren 19,4 ml einer 1,6 molaren n-Butyllithiumlösung in n-Hexan (31 mMol) zutropfen, rührt noch 1 Stunde bei -78 °C nach, fügt rasch 7,45 g (54,6 mMol) Trifluormethansulfenylchlorid zu und läßt dann auf Raumtemperatur erwärmen.

Nach Zugabe von 100 ml Wasser wird nochmals mit je 50 ml Ether extrahiert und nach dem Trocknen über Natriumsulfat und Einengen auf ca. 20 ml über eine mit Kieselgel beschickte Säule mit Ether filtriert.

Man erhält 7,2 g (88 % der Theorie) 2-Trifluormethylthio-4-(2′-fluorphenyl)-1,3-oxazol als gelbes Öl. ($^1$H-NMR ($CDCl_3$); $\delta$ = 8,25-7,0 (m; 5H, Phenyl-H, 5-H)

### Beispiel 2

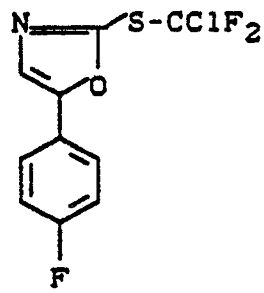

Unter Argon-Atmosphäre läßt man zur Lösung von 8,9 g (55 mMol) 5-(4′-fluorphenyl)-1,3-oxazol in 100 ml wasserfreiem Tetrahydrofuran bei -78° C unter Rühren 36 ml einer 1,6 molaren n-Butyllithium-Lösung in n-Hexan (58 mMol) zutropfen, rührt noch 1 Stunde bei -78° C nach, fügt rasch 8,8 g (58 mMol) Chlordifluormethansulfenylchlorid zu und läßt dann auf Raumtemperatur erwärmen.

Nach Zugabe von 100 ml Wasser wird mehrmals mit je 50 ml Ether extrahiert und nach dem Trocknen über Natriumsulfat und Einengen auf ca. 20 ml über eine mit Kieselgel beschickte Säule mit Ether filtriert. Man erhält 7,5 g (49 % der Theorie) 2-Chlor-difluormethylthio-5-(4′-fluorphenyl)-1,3-oxazol von Schmelzpunkt 52-54° C.

Analog Beispiel 1 und 2 bzw. dem erfindungsgemäßen Verfahren können die in der nachfolgenden Tabelle 1 angegebenen Verbindungen der Formel (I) hergestellt werden:

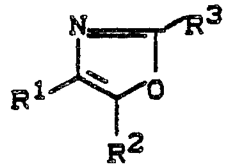

(I)

Tabelle : 1

| Bsp. Nr. | $R^1$ | $R^2$ | $R^3$ | physikal. Konstante |
|---|---|---|---|---|
| 3 | H | 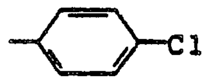 | $SCCl_2F$ | Fp: 76° C |
| 4 | H | 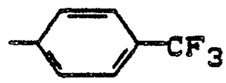 | $SCCl_2F$ | |
| 5 | H | 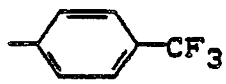 | $SCClF_2$ | |
| 6 | $CH_3$ | 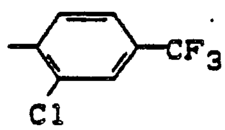 | $SCClF_2$ | |
| 7 | H | 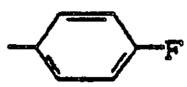 | $SCCl_2F$ | |
| 8 | 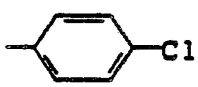 | H | $SCClF_2$ | Fp:47° C |
| 9 | 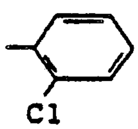 | H | $SCClF_2$ | |
| 10 | 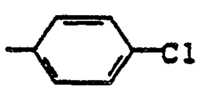 | H | $SCF_3$ | |

Tabelle : 1 - Fortsetzung

| Bsp. Nr. | $R^1$ | $R^2$ | $R^3$ | physikal. Konstante |
|---|---|---|---|---|
| 11 | 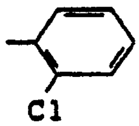 | H | $SCF_3$ | |
| 12 | 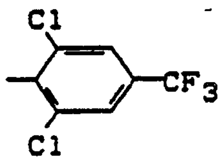 | $CH_3$ | $SCF_3$ | |
| 13 | 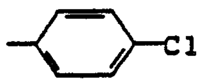 | H | $SCCl_2F$ | Fp:79° C |
| 14 | 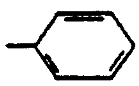 | H | $SCCl_2F$ | Fp:73° C |
| 15 | 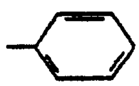 | H | $SCClF_2$ | Fp:59° C |
| 16 | 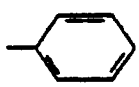 | H | $SCF_3$ | Fp:46° C |
| 17 | 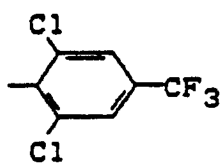 | H | $SCF_3$ | Fp:62° C |
| 18 | H | 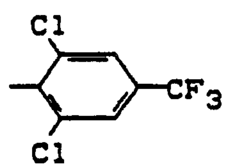 | $SCClF_2$ | |
| 19 | H | 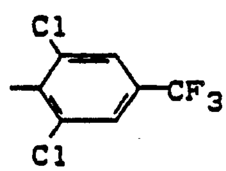 | $SCF_3$ | |

Tabelle : 1 - Fortsetzung

| Bsp. Nr. | $R^1$ | $R^2$ | $R^3$ | physikal. Konstante |
|---|---|---|---|---|
| 19 | H | 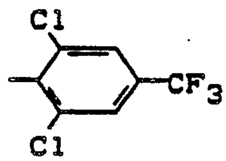 | $SCF_3$ | |
| 20 | 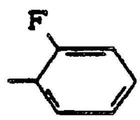 | H | $SCClF_2$ | |
| 21 | 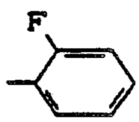 | H | $SCCl_2F$ | Fp:46-48° C |
| 22 | 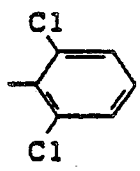 | H | $SCCl_2F$ | Fp:68-70° C |
| 23 | 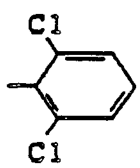 | H | $SCClF_2$ | Fp:54-56° C |
| 24 | 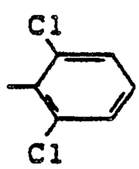 | H | $SCF_3$ | |
| 25 | 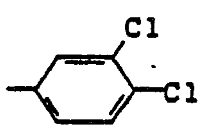 | H | $SCClF_2$ | |

Tabelle : 1 - Fortsetzung

| Bsp. Nr. | $R^1$ | $R^2$ | $R^3$ | physikal. Konstante |
|---|---|---|---|---|
| 26 | 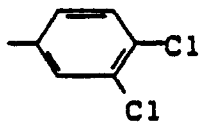 | H | $SCCl_2F$ | |
| 27 | 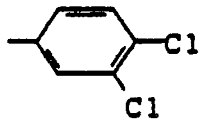 | H | $SCF_3$ | |
| 28 | 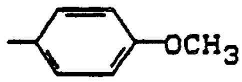 | H | $SCClF_2$ | |
| 29 | 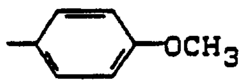 | H | $SCCl_2F$ | |
| 30 | 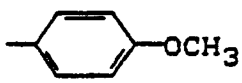 | H | $SCF_3$ | |
| 31 | 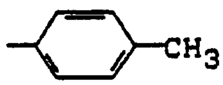 | H | $SCClF_2$ | Fp:58-59° |
| 32 | 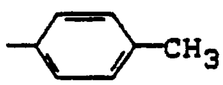 | H | $SCCl_2F$ | |
| 33 | 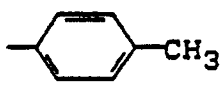 | H | $SCF_3$ | |
| 34 | 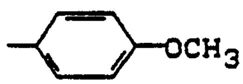 | H | $SCH_3$ | |
| 35 | H | 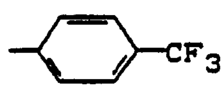 | $SCH_3$ | |
| 36 | 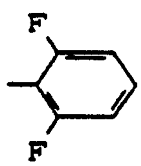 | H | $SCH_3$ | |

Tabelle : 1 - Fortsetzung

| Bsp. Nr. | $R^1$ | $R^2$ | $R^3$ | physikal. Konstante |
|---|---|---|---|---|
| 37 | 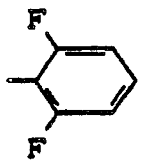 | H | $SCF_2Cl$ | |
| 38 | 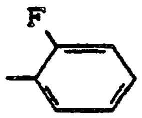 | H | $SCH_3$ | |
| 39 | 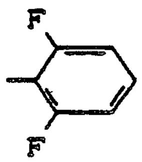 | H | $SCF_3$ | |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Verwendungsbeispiele

In den folgenden Vergleichsbeispielen wird die nachstehend aufgeführte Verbindung als Vergleichssubstanz eingesetzt:

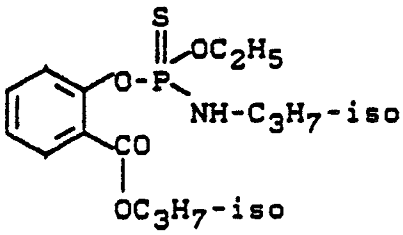

(A)

O-Ethyl-O-(2-isopropoxycarbonyl-phenyl)-N-isopropylthionophosphorsäureesteramid (bekannt aus DE-OS 16 68 047)

Beispiel A

Grenzkonzentrations-Test

| | |
|---|---|
| Testnematode: | Meloidogyne incognita |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, pflanzt Kartoffeln ein und hält die Töpfe bei einer Gewächshaus-Temperatur von 18 °C.

Nach sechs Wochen werden die Kartoffelwurzeln auf Zysten untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele 1, 2 und 5 überlegene Wirksamkeit gegenüber dem Stand der Technik.

Beispiel B

Grenzkonzentrations-Test

| | |
|---|---|
| Testnematode: | Globodera rostochiensis |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den

behandelten Boden in Töpfe, pflanzt Kartoffeln ein und hält die Töpfe bei einer Gewächshaus-Temperatur von 18 °C.

Nach sechs Wochen werden die Kartoffelwurzeln auf Zysten untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele 1 und 20 überlegene Wirksamkeit gegenüber dem Stand der Technik.

## Ansprüche

1. 2-(Halogen)alkylthio-1,3-oxazol-Derivate der Formel

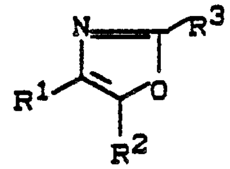

(I)

in welcher
$R^1$ für Wasserstoff, Alkyl oder gegebenenfalls substituiertes Phenyl steht,
$R^2$ für Wasserstoff, Alkyl oder gegebenenfalls substituiertes Phenyl steht und
$R^3$ für gegebenenfalls durch Halogen substituiertes Alkylthio steht,
mit der Maßgabe, daß $R^1$ und $R^2$ voneinander verschieden sind.

2. 2-(Halogen)alkylthio-1,3-oxazol-Derivate der Formel (I) gemäß Anspruch 1,
in welcher
$R^1$ für Wasserstoff, ($C_1$-$C_4$)-Alkyl oder für gegebenenfalls einfach bis fünffach, gleich oder verschieden durch Fluor, Chlor, Brom, durch ($C_1$-$C_4$)-Alkyl (welches gegebenenfalls durch Fluor und/oder Chlor substituiert ist) oder durch ($C_1$-$C_4$)-Alkoxy substituiertes Phenyl steht,
$R^2$ für Wasserstoff, ($C_1$-$C_4$)-Alkyl oder für gegebenenfalls einfach bis fünffach, gleich oder verschieden durch Fluor, Chlor, Brom, durch ($C_1$-$C_4$)-Alkyl (welches gegebenenfalls durch Fluor und/oder Chlor substituiert ist) oder durch ($C_1$-$C_4$)-Alkoxy substituiertes Phenyl steht und
$R^3$ für gegebenenfalls durch Fluor und/oder Chlor substituiertes ($C_1$-$C_4$)-Alkylthio steht.

3. 2-(Halogen)alkylthio-1,3-oxazol-Derivate der Formel (I) gemäß Anspruch 1,
in welcher
$R^1$ für Wasserstoff, methyl, Ethyl oder für gegebenenfalls einfach bis dreifach, gleich oder verschieden durch Fluor und/oder Chlor, durch ($C_1$-$C_2$)-Alkyl (welches gegebenenfalls einfach bis fünffach durch Fluor und/oder Chlor substituiert ist), oder durch Methoxy oder Ethoxy substituiertes Phenyl steht,
$R^2$ für Wasserstoff, Methyl, Ethyl oder für gegebenenfalls einfach bis dreifach, gleich oder verschieden durch Fluor und/oder Chlor, durch ($C_1$-$C_2$)-Alkyl (welches gegebenenfalls einfach bis fünffach durch Fluor und/oder Chlor substituiert ist), oder durch Methoxy oder Ethoxy substituiertes Phenyl steht und
$R^3$ für gegebenenfalls einfach bis fünffach, gleich oder verschieden durch Fluor und/oder Chlor substituiertes ($C_1$-$C_2$)-Alkylthio steht.

4. 2-(Halogen)alkylthio-1,3-oxazol-Derivate der Formel (I) gemäß Anspruch 1, in welcher
$R^1$ für Wasserstoff, Methyl oder für gegebenenfalls einfach bis dreifach, gleich oder verschieden durch Fluor und/oder Chlor, Trifluormethyl oder Methoxy substituiertes Phenyl steht,
$R^2$ für Wasserstoff, Methyl oder für gegebenenfalls einfach bis dreifach, gleich oder verschieden durch Fluor und/oder Chlor, Trifluormethyl oder Methoxy substituiertes Phenyl steht und
$R^3$ für Methylthio, Chlordifluormethylthio, Dichlorfluormethylthio oder Trifluormethylthio steht.

5. Verfahren zur Herstellung von 2-(Halogen)alkylthio-1,3-oxazol-Derivaten der Formel

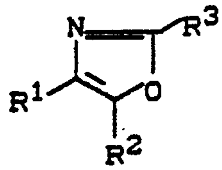

(I)

in welcher
$R^1$ für Wasserstoff, Alkyl oder gegebenenfalls substituiertes Phenyl steht,
$R^2$ für Wasserstoff, Alkyl oder gegebenenfalls substituiertes Phenyl steht und
$R^3$ für gegebenenfalls durch Halogen substituiertes Alkylthio steht,
mit der Maßgabe, daß $R^1$ und $R^2$ voneinander verschieden sind,
dadurch gekennzeichnet, daß man 1,3-Oxazol-Derivate der Formel

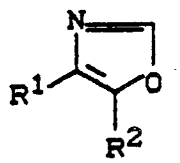

(II)

in welcher
$R^1$ und $R^2$ die oben angegebene Bedeutung besitzen,
mit Lithiumverbindungen der Formel
$R^4$-Li (III)
in welcher
$R^4$ für Methyl oder n-Butyl steht,
gegebenenfalls in Gegenwart von Verdünnungsmitteln, gegebenenfalls in Gegenwart eines Inertgases und anschließend mit Sulfenylhalogeniden der Formel
$R^3$ - X (IV)
in welcher
$R^3$ für gegebenenfalls durch Halogen substituiertes Alkylthio steht und
X für Halogen steht,
umsetzt.

6. Schädlingsbekämpfungsmittel, gekennzeichnet durch einen Gehalt an mindestens einem 2-(Halogen)alkylthio-1,3-oxazol-Derivat der Formel (I).

7. Insektizides Mittel, gekennzeichnet, durch einen Gehalt an mindestens einem 2-(Halogen)alkylthio-1,3-oxazol-Derivat der Formel (I).

8. Verfahren zur Bekämpfung von Insekten, dadurch gekennzeichnet, daß man 2-(Halogen)alkylthio-1,3-oxazol-Derivate der Formel (I) auf Insekten und/oder deren Lebensraum einwirken läßt.

9. Verwendung von 2-(Halogen)alkylthio-1,3-oxazol-Derivaten zur Bekämpfung von Insekten.

10. Verfahren zur Herstellung von Schädlingsbekämpfungsmitteln, dadurch gekennzeichnet, daß man 2-(Halogen)alkylthio-1,3-oxazol-Derivate der Formel (I) mit Streckmitteln und/oder oberflächenaktiven Mitteln vermischt.